(19) Europäisches Patentamt
European Patent Office
Office européen des brevets

(11) Publication number: **0 116 238**
**A1**

(12) **EUROPEAN PATENT APPLICATION**

(21) Application number: 83308041.9

(22) Date of filing: 30.12.83

(51) Int. Cl.³: A 61 K 31/40
A 61 K 45/06
//(A61K31/40, 31/13)

(30) Priority: 05.01.83 US 455808
16.12.83 US 561351

(43) Date of publication of application:
22.08.84 Bulletin 84/34

(84) Designated Contracting States:
AT BE CH DE FR GB IT LI LU NL SE

(71) Applicant: BIOMEASURE INC.
11-15 E. Avenue
Hopkinton Massachusetts(US)

(72) Inventor: Moreau, Jacques-Pierre
159 Westboro Road
Upton Massachusetts(US)

(74) Representative: Deans, Michael John Percy et al,
Lloyd Wise, Tregear & CO. Norman House 105-109 Strand
London WC2R OAE(GB)

(54) **Therapeutic compositions and their use in enhancing brain function.**

(57) The use conjointly of a piracetam analog capable of enhancing firing of acetylcholine-containing brain neurons in a human while depleting the brain acetylcholine level, and a second compound capable of slowing the rate of choline depletion in a human, is described for the therapeutic enhancement of brain function.

EP 0 116 238 A1

Croydon Printing Company Ltd

# Therapeutic Compositions and their use in enhancing brain function

This invention relates to brain-function enhancing compositions.

Various drugs are known for the treatment of medical disorders affecting acetylcholine-containing neurons in the brain. One such drug, piracetam, has been administered alone and, as is described in Growdon et al. U.S. Patent No. 4,355,927, in combination with choline, choline salts, "or a compound that dissociates to choline, such as an acylglycerophosphocholine, e.g., lecithin, lysolecithin, [or] glycerophosphatidyl choline."

Piracetam has the following structure:

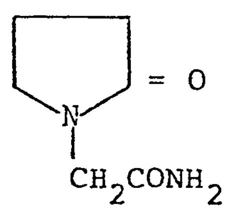

A number of analogs of piracetam are known which, like piracetam, are capable of enhancing the firing of acetylcholine-containing brain neurons in a human, while depleting brain acetylcholine level.

One such analog is oxiracetam, having the structure:

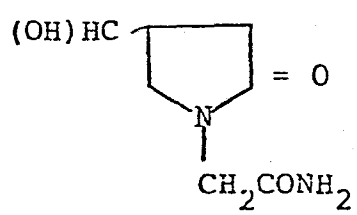

Oxiracetam is described in Itil et al. (1982) Drug Development Research 2, 447.

Another piracetam analog is etiracetam, having the structure:

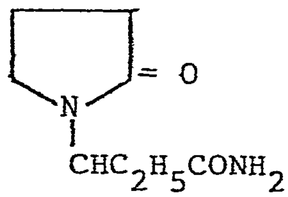

Etiracetam is described in British Pat. No. 1,309,692.

Another piracetam analog is pramiracetam (usually provided in the form of the hydrochloride), having the structure:

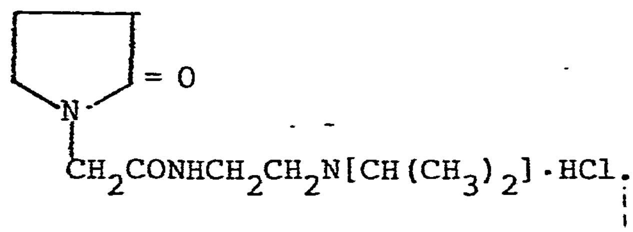

Pramiracetam is described in British Pat. No. 1,588,082.

Another piracetam analog, aniracetam, is described in Foltyn et al. (1983) Drug Res. 33(I), 865.

General formulae including additional effective piracetam analogs are disclosed, e.g., in British Pat. No. 1,588,082.

Another drug which has been described in the literature is deanol, described in Harbrich et al. (1981) J. Neurochem. 37(2), 476:

> [T]he central cholinergic actions of deanol could result from an increase in the concentration of free choline in blood, which in turn stimulates cholinoceptive neurons in the brain. The relatively long duration of action of deanol (several hours) in

producing an increase in peripheral concentration of choline, as compared with the increase caused by parenteral administration of choline (approximately 20 minutes), suggests that treatment of patients with deanol or a combination of choline plus deanol may be preferable to high doses of choline as a means of raising the levels of choline in the body. The usefulness of choline as a therapeutic agent is limited by its relatively rapid removal from the body and by its metabolism in the gut to trimethylamine, which leads to an undesirable odor in treated patients. The present results suggest that an inhibitor of choline metabolism in the periphery may be useful when given alone, or in combination with choline, to elicit a central cholinergic effect.

In general, the invention features enhancing brain function in a human by administering to the human an effective amount of a piracetam analog capable of enhancing firing of acetylcholine-containing brain neurons in a human while depleting the brain acetylcholine level in the human, and an effective amount of a second compound capable of slowing the rate of choline depletion in the human.

Preferably the second compound is present in an amount effective to slow choline depletion to a degree sufficient to counteract the acetylcholine depletion caused by the piracetam analog; i.e., to prevent the piracetam analog from bringing about a net decrease in brain acetylcholine.

In preferred embodiments the piracetam analog is piracetam, aniracetam, pramiracetam, etiracetam, or oxiracetam; and the second compound is deanol or a

compound which dissociates to form deanol, e.g., a salt or ester of deanol, e.g., meclofenoxate or pyrisuccideanol.

In other preferred embodiments, the second compound is an acetylcholine esterase inhibitor, e.g. physostigmine.

Two such second compounds can be used together, e.g., deanol and physostigmine can be used together. The advantage of such use is that, for example, when deanol and physostigmine are both used, lower amounts of each can be used; this is particularly advantageous in the case of physostigmine, which is toxic in large doses.

The invention provides the brain function enhancement of piracetam analogs without the disadvantages associated with these. Piracetam analogs work by enhancing firing of brain neurons. This firing enhancement can deplete brain acetylcholine levels, thereby producing, in one respect, an effect deleterious to brain function. According to the invention, this deleterious effect is counteracted by a compound capable of slowing the rate of choline depletion; e.g., deanol, which inhibits choline metabolism.

Other features and avantages of the invention will be apparent from the following description.

## Description of Preferred Embodiments

### Composition

The preferred therapeutic composition contains piracetam and deanol or a compound which dissociates to form deanol. Both piracetam and deanol are commercially available, as are deanol esters and salts such as meclofenoxate (Fr. Pat. M398) and pyrisuccideanol (German Pat. No. 2,102,831).

Use

The piracetam analog and the second compound can each be administered alone or in combination with a pharmaceutically acceptable carrier, or the active agents and carrier can be combined.

For oral administration the pharmaceutical compositions can be provided in the form of capsules or tablets, either ordinary or time-release. The compositions can also take the form of ingestible liquid, e.g., syrup, or an injectible liquid.

The piracetam analog can be administered in an amount between 0.1 and 100 mg/kg/day. The second compound, e.g. deanol or a compound which dissociates to form deanol, can be administered in an amount between 10 and 2,000 mg/kg/day.

The piracetam analog and second compound, rather than being administered in one therapeutic composition, can be administered simultaneously or near enough in time to achieve the desired counteracting effect.

When administered to a human (e.g., orally, parenterally, intravenously, or by suppository) the compositions of the invention can enhance brain function associated with acetylcholine-containing brain neurons.

0116238

## Claims

1. A therapeutic composition comprising a therapeutically effective amount of a piracetam analog capable of enhancing firing of acetylcholine-containing brain neurons in a human while depleting the brain acetylcholine level in said human, and

a therapeutically effective amount of a second compound capable of slowing the rate of choline depletion in said human.

2. A composition according to Claim 1, wherein the relative proportion of the second compound in said composition is such as to be effective to slow such depletion to a degree sufficient to counteract the said depletion caused by said piracetam analog.

3. A composition according to Claim 1 or 2, wherein said piracetam analog is piracetam, aniracetam, pramiracetam, etiracetam, or oxiracetam.

4. A composition according to any preceding claim, wherein said second compound is deanol or a compound which dissociates to form deanol.

5. A composition according Claim 4, further comprising an effective, non-toxic amount of an acetylcholine esterase inhibitor.

6. A composition according to any of Claims 1 to 3, wherein said second compound is an acetylcholine esterase inhibitor.

7. A composition according to Claims 5 or 6 wherein said acetylcholine esterase inhibitor is physostigmine.

8. A composition according to Claims 4 or 5 wherein said compound which dissociates to form deanol is an ester of deanol, a salt of deanol, meclofenoxate or pyrisuccideanol.

9. The use conjointly in human therapy, for enhancing brain function, of

(i) a piracetam analog capable of enhancing firing of acetylcholine containing brain neurons while depleting the brain acetylcholine level, and

(ii) a second compound capable of slowing the rate of choline depletion in said human.

10. The use conjointly of a piracetam analog and a second compound according to Claim 9, wherein the piracetam analog and said second compound are administered simultaneously or near enough in time so that said second compound counteracts the effect of said piracetam analog.

11. The conjoint use according to Claims 9 or 10 of a piracetam analog and a second compound, wherein said piracetam analog is administered in an amount between 0.1 and 100 mg/kg/day and said second compound is administered in an amount between 10 and 2,000 mg/kg/day.

12. The conjoint use according to Claims 9 or 10 of a piracetam analog and a second compound, wherein said second compound is administered in an amount effective

to slow such depletion to a degree sufficient to counteract the said depletion caused by said piracetam analog.

13. The use conjointly in human therapy, for enhancing brain function, of piracetam and of a second compound capable of slowing the rate of choline depletion in said human.

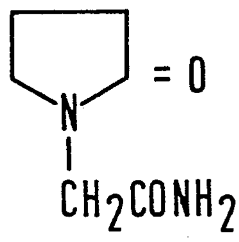

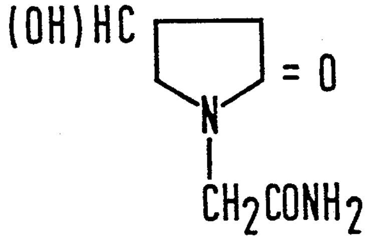

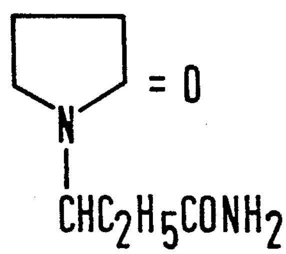

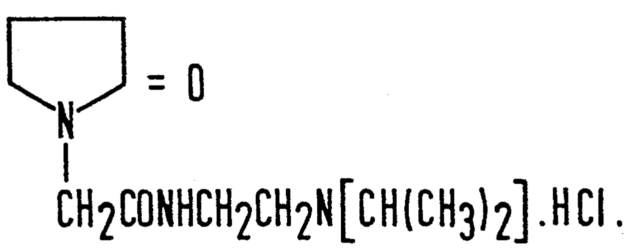

0116238

European Patent Office

# PARTIAL EUROPEAN SEARCH REPORT

which under Rule 45 of the European Patent Convention shall be considered, for the purposes of subsequent proceedings, as the European search report

Application number: EP 83 30 8041

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | UNLISTED DRUGS, vol. 33, no. 12, December 1981, Chatham, New Jersey, US<br>* Page 191m "Centracetam" *<br>-- | 1-8 | A 61 K 31/40<br>A 61 K 45/06//<br>(A 61 K 31/40<br>31/13) |
| AD | US - A - 4 355 027 (JOHN H. CROWDON)<br>----- | 1-8 | |

TECHNICAL FIELDS SEARCHED (Int. Cl. 3)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-8

Claims searched incompletely:

Claims not searched: 9-13

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 11-04-1984 | BRINKMANN |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82